(19) Europäisches Patentamt
European Patent Office
Office européen des brevets

(11) Veröffentlichungsnummer: **0 183 959**
**B1**

(12) # EUROPÄISCHE PATENTSCHRIFT

(45) Veröffentlichungstag der Patentschrift: **15.02.89**

(21) Anmeldenummer: **85113421.3**

(22) Anmeldetag: **23.10.85**

(51) Int. Cl.[4]: **C 07 C 131/00**, C 07 C 62/38, C 07 C 49/573, C 07 C 121/34, A 01 N 35/06, A 01 N 37/44

(54) **Cyclohexenonderivate, Verfahren zu ihrer Herstellung sowie ihre Verwendung zur Bekämpfung unerwünschten Pflanzenwuchses.**

(30) Priorität: **06.11.84 DE 3440410**

(43) Veröffentlichungstag der Anmeldung: **11.06.86 Patentblatt 86/24**

(45) Bekanntmachung des Hinweises auf die Patenterteilung: **15.02.89 Patentblatt 89/7**

(84) Benannte Vertragsstaaten: **BE CH DE FR GB IT LI NL**

(56) Entgegenhaltungen:
**EP-A-0 066 588**
**EP-A-0 095 099**
**GB-A-2 077 732**

**CHEMICAL ABSTRACTS, Band 86, Nr. 25, 20. Juni 1977, Seite 558, Spalte 1, Zusammenfassungsnr. 189318z, Columbus, Ohio, US; & JP - A - 76 131 856 (NIPPON SODA CO.) 16.11.1976**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Becker, Rainer, Dr., Im Haseneck 22, D-6702 Bad Duerkheim (DE)**
Erfinder: **Jahn, Dieter, Dr., Burgunder Weg 8, D-6803 Edingen- Neckarhausen (DE)**
Erfinder: **Schirmer, Ulrich, Dr., Berghalde 79, D-6900 Heidelberg (DE)**
Erfinder: **Keil, Michael, Dr., Fontanestrasse 4, D-6713 Freinsheim (DE)**
Erfinder: **Wuerzer, Bruno, Dr., Ruedigerstrasse 13, D-6701 Otterstadt (DE)**
Erfinder: **Meyer, Norbert, Dr., Dossenheimer Weg 22, D-6802 Ladenburg (DE)**

EP 0 183 959 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft Cyclohexenonderivate, Verfahren zu ihrer Herstellung, Herbizide, die diese Verbindungen als Wirkstoffe enthalten sowie ein Verfahren zur Bekämpfung von unerwünschtem Pflanzenwuchs.

Es ist bekannt, daß Cyclohexenonderivate herbizid wirksam sind (DE-A-2 439 104, JP-A-19 945/1979).

Es wurde gefunden, daß Cyclohexenonderivate der Formel I

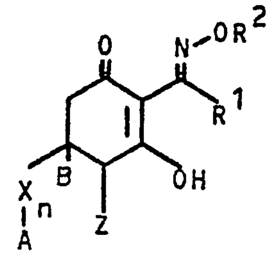

(I),

in der

R1 $C_1$-$C_4$-Alkyl,

$R^2$ $C_1$-$C_4$-Alkyl, gegebenenfalls halogensubstituiertes $C_3$-$C_5$-Alkenyl oder $C_3$-$C_5$-Alkinyl,

A $C_2$-$C_5$-Alkoxycarbonyl, Carboxyl, Cyano oder Trifluormethyl,

B Wasserstoff oder Methyl,

X einen unverzweigten, verzweigten oder cyclischen Alkylenrest mit bis zu 7 C-Atomen,

Z Wasserstoff oder $C_2$-$C_5$-Alkoxycarbonyl und

n 0 oder 1 bedeuten,

mit der Maßgabe, daß n nicht 0 bedeutet, wenn A für Alkoxycarbonyl oder Cyano steht, sowie Salze dieser Verbindungen eine gute herbizide Wirkung vorzugsweise gegen Arten aus der Familie der Gräser (Gramineen) besitzen. Sie sind verträglich und somit selektiv in breitblättrigen Kulturen sowie in monokotylen Gewächsen, welche nicht zu den Gramineen zählen, bzw. selektiv in Gramineenkulturen, wie Weizen und Reis, und gleichzeitig herbizid für unerwünschte Gräser.

Die Cyclohexenonderivate der Formel I können in tautomeren Formen auftreten, die alle vom Patentanspruch umfaßt werden:

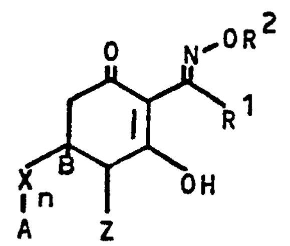

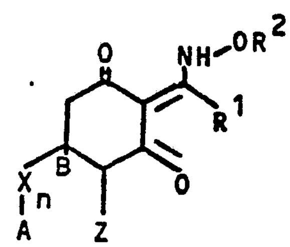

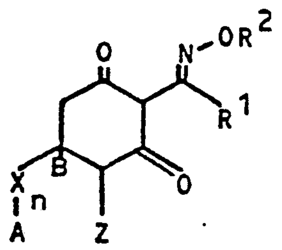

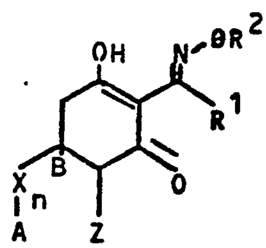

Die Substitutenten in Formel I können folgende Bedeutungen haben:

$R^1$ steht für unverzweigtes oder verzweigtes $C_1$-$C_4$-Alkyl, vorzugsweise $C_2$- und $C_3$-Alkyl, beispielsweise Methyl, Ethyl, n-Propyl, s-Butyl oder n-Butyl.

$R^2$ bedeutet unverzweigtes oder verzweigtes $C_1$-$C_4$-Alkyl, $C_3$-$C_5$-Alkenyl, das durch Halogen, insbesondere Chlor, substituiert sein kann, oder $C_3$-$C_5$-Alkinyl, beispielsweise Methyl, Ethyl, n-Propyl, n-Butyl, Allyl, Propargyl, 2-Chlorallyl oder 3-Chlorallyl (cis oder trans).

A steht für $C_2$-$C_5$-Alkoxycarbonyl, beispielsweise Methoxycarbonyl oder Ethoxycarbonyl, sowie für Carboxyl,

Cyano oder Trifluormethyl.

B bedeutet Wasserstoff oder Methyl, wobei Wasserstoff bevorzugt ist.

X bedeutet einen unverzweigten oder verzweigten Alkylenrest mit bis zu 7 C-Atomen oder einen cyclischen Alkylenreste mit 3 bis 7 C-Atomen, beispielsweise Methylen, Ethylen, Trimethylen, Tetramethylen, Methylmethylen, Methylethylen, 2,5-Dimethyl-pentamethylen, 1,4-Cyclohexylen, 1,3-Cyclohexylen, 1,2-Cyclohexylen oder 1,2-Cyclopropylen.

Z steht schließlich für Wasserstoff oder $C_2$-$C_5$-Alkoxycarbonyl, wie Methoxycarbonyl oder Ethoxycarbonyl, wobei Methoxycarbonyl und insbesondere Wasserstoff bevorzugt sind.

Als Salze der Verbindungen der Formel I kommen landwirtschaftlich brauchbare Salze, beispielsweise die Alkalimetallsalze, insbesondere die Kalium- oder Natriumsalze, Erdalkalimetallsalze, insbesondere Calcium-, Magnesium- oder Bariumsalze, Mangan-, Kupfer-, Zink- oder Eisensalze sowie Ammonium-, Sulfonium- und Phosphoniumsalze in Betracht,

Man erhält die neuen Cyclohexenonderivate der Formel I durch Umsetzung der ebenfalls neuen Carbonylverbindungen der Formel II

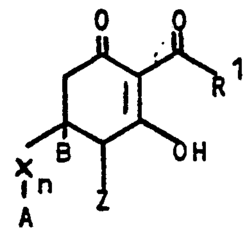

(II),

in der $R^1$, A, B, Z, X und n die jeweils oben genannte Bedeutung haben, mit Hydroxylaminderivaten der Formel

$R^2ONH_3Y$,
in der $R^2$ die obengenannte Bedeutungen besitzt und
Y ein Anion, z. B. Chlorid, Bromid oder Sulfat,

bedeutet.

Man führt die Umsetzung zweckmäßigerweise in heterogener Phase in einem inerten Verdünnungsmittel bei einer Temperatur im Bereich zwischen o und ungefährt 80°C oder zwischen 0°C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium oder Calcium. Außerdem können auch organische Basen, wie Pyridin oder tertiäre Amine, Verwendung finden.

Die Umsetzung verläuft gut in einem pH-Bereich von 2 bis 9, vorzugsweise 4 bis 6, insbesondere 4,5 bis 5,5. Die Einstellung des pH-Bereichs erfolgt zweckmäßig durch Zusatz von Acetat, beispielsweise Alkalimetallacetat, insbesondere von Natrium- oder Kaliumacetat oder einer Mischung aus beiden Salzen. Alkalimetallacetat wird beispielsweise in Megen von 0,5 bis 2 Mol, bezogen auf 1 Mol Ammoniumverbindung der Formel $R^2ONH_3Y$, zugesetzt.

Als Lösungsmittel sind beispielsweise Dimethylsulfoxid; Alkohole, wie Methanol, Ethanol oder Isopropanol; Benzol; gegebenenfalls chlorierte Kohlenwasserstoffe, wie Chloroform, Dichlorethan, Hexan oder Cyclohexan; Ester, wie Essigsäureethylester; oder cyclische Ether, wie Dioxan oder Tetrahydrofuran, geeignet.

Die Umsetzung ist nach wenigen Stunden beendet, das Reaktionsprodukt kann dann durch Einengen des Reaktionsgemisches, Zugabe von Wasser und Extraktion mit einem unpolaren Lösungsmittel, wie Methylenchlorid und Abdestillieren des Lösungsmittels unter vermindertem Druck isoliert werden.

Die Verbindungen der Formel I können außerdem durch Umsetzung der Carbonylverbindungen der Formel II mit Hydroxylaminen der Formel

$R^2ONH_2$,

in der $R^2$ die obengenannte Bedeutung hat, in inerten Verdünnungsmitteln bei einer Temperatur im Bereich zwischen 0°C und dem Siedepunkt des Reaktionsgemisches erhalten werden. Zweckmäßig erhitzt man auf eine Temperatur von ca. 15 bis 70°C. Gegebenenfalls kann das Hydroxylamin auch als wäßrige Lösung eingesetzt werden.

Geeignete Lösungsmittel für diese Umsetzung sind beispielsweise Alkohole, wie Methanol, Ethanol, Isopropanol oder Cyclohexanol; gegebenenfalls chlorierte Kohlenwasserstoffe, wie Hexan, Cyclohexan, Methylenchlorid, Toluol oder Dichlorethan; Ester, wie Essigsäureethylester; Nitrile, wie Acetonitril; oder cyclische Ether, wie Tetrahydrofuran.

Schließlich können die Carbonylverbindungen der Formel II auch mit einem unsubstituierten Hydroxylammoniumsalz der Formel

$HONH_3Y$, in der

Y ein Anion bedeutet (z. B. Chlorid, Bromid oder Sulfat), in Gegenwart eines Lösungsmittels und einer Base umgesetzt werden, wobei die Reaktionsbedingungen analog denen der obengenannten Reaktion mit der Ammoniumverbindung der Formel $R^2ONH_3Y$ sind. Anschließend wird dann das so erhaltene Oxim mit einem Alkylierungsmittel der Formel

$R^2Y'$,
in der $R^2$ die obengenannte Bedeutung besitzt und
Y' eine Abgangsgruppe (z. B. Chlor, Brom, Jod oder $R^2OSO_3$) bedeutet,
bei einer Temperatur von 0 bis 100°C in einem inerten Lösungsmittel, wie Dioxan, Tetrahydrofuran oder N,N-Dimethylformamid, gegebenenfalls in Gegenwart einer Base (vgl. die obengenannten Basen) in die Zielverbindung der Formel I übergeführt.

Die Alkalimetallsalze der Cyclohexenonderivate der Formel I können durch Behandeln dieser Verbindungen mit Natrium- oder Kaliumhydroxid in wäßriger Lösung oder in einem organischen Lösungsmittel, wie Methanol, Ethanol oder Aceton, erhalten werden. Auch Natrium- und Kaliumalkoholate können zur Salzbildung verwendet werden.

Die anderen Metallsalze, z. B. die Mangan-, Kupfer-, Zink-, Eisen-, Calcium-, Magnesium- und Bariumsalze, können aus den Natriumsalzen durch Reaktion mit den entsprechenden Metallchloriden in wäßriger Lösung hergestellt werden. Ammonium-, Sulfonium- und Phosphoniumsalze können durch Umsetzung von Vorbindungen der Formel I mit Ammonium-, Sulfonium- oder Phosphoniumhydroxiden gegebenenfalls in wäßriger Lösung hergestellt werden.

Die erfindungsgemäßen Carbonylverbindungen der Formel II können aus Cyclohexandionen der Formel III, die auch in der tautomeren Form IIIa vorliegen können,

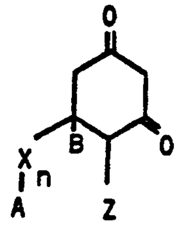

(III)

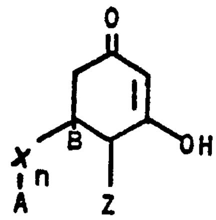

(IIIa)

nach literaturbekannten Methoden (Tetrahedron Letters 29, (1975) 2491) hergestellt werden.

Es ist auch möglich, die Carbonylverbindungen der Formel II über die Zwischenstufe der Enolester IV, die bei der Umsetzung der Derivate III mit Säurechloriden anfallen und in Gegenwart von Imidazol- oder Pyridinderivaten umgelagert werden, herzustellen (JP-A-63 052/1979)

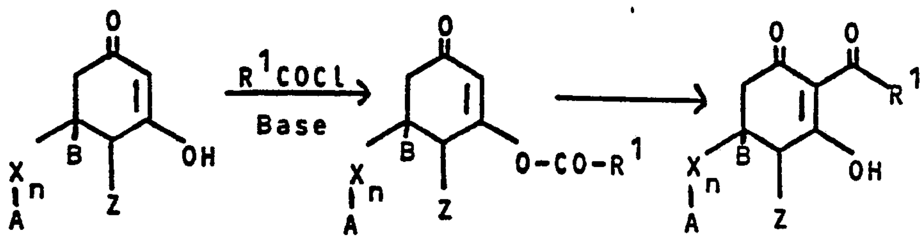


(III) (IV) (II)

Zu den Verbindungen der Formel III gelangt man nach an sich bekannten Verfahren (Org, Synth. Coll. II, Seite 200). Neben der dort beschriebenen alkalischen Verseifung mit anschließender saurer Decarboxylierung kann die Abspaltung der aktivierten Estergruppe auch in neutralem Medium mit Dimethylsulfoxid/Wasser/Natriumchlorid erfolgen (Synthesis 1982, 805), was vor allem bei Vorhandensein weiterer labiler Gruppen im Molekül zweckmäßig ist.

Wie sich aus dem Voranstehenden ergibt, stellen die erfindungsgemäßen Carbonylverbindungen der Formel II wertvolle Zwischenprodukte für die Synthese der herbizid wirksamen Cyclohexenonderivate der Formel I dar.

Die folgenden Beispiele erläutern die Herstellung der erfindungsgemäßen Cyclohexenonderivate der Formel I:

**Beispiel 1**

9,21 g 2-Butyryl-5-(4-trifluormethylcyclohexyl)-cyclohexan-1,3-dion wurden in 100 ml Methanol aufgenommen und mit 2,94 g Ethoxyammoniumchlorid sowie 2,52 g Natriumyhdrogencarbonat versetzt und 20 Stunden lang bei Raumtemperatur gerührt. Anschließend gab man in Wasser, extrahierte mit Methylenchlorid und engte ein. Man erhielt 7,9 g 2-(1-Ethoxyamino-butyliden)-5-(4-trifluormethylcyclohexyl)-cyclohexan-1,3-dion (Verbindung Nr. 1).

$^1$H-NMR (in $CDCl_3$/TMS) δ = 4,08 (q) [2 Protonen]
1,90 (m) [Cyclohexylprotonen]
0,92 (t) [3 Protonen]

Die folgenden Cyclohexenonderivate der Formel I

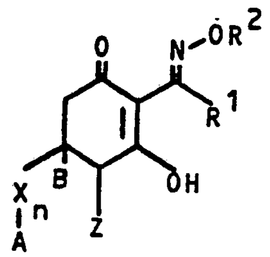

(I)

lassen sich auf analogem Wege herstellen.

Bei den in der folgenden Tabelle 1 aufgeführten Substituenten X soll jeweils deren linke Bindung an den Cyclohexanring gebunden sein.

**Tabelle 1**

| Verbindung Nr. | A | X | n | B | Z | $R^1$ | $R^2$ | phys. Daten | |
|---|---|---|---|---|---|---|---|---|---|
| 2 | $CF_3$ | 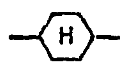 | 1 | H | H | n-$C_3H_7$ | $CH_2CH=CH_2$ | $n_D^{22}$ | 1,4941 |
| 3 | $COOCH_3$ | $-(CH_2)_4-$ | 1 | H | $COOCH_3$ | n-$C_3H_7$ | $C_2H_5$ | $n_D^{25}$ | 1,5067 |
| 4 | $COOCH_3$ | $-(CH_2)_4-$ | 1 | H | $COOCH_3$ | n-$C_3H_7$ | $CH_2CH=CH_2$ | $n_D^{25}$ | 1,5108 |
| 5 | $COOCH_3$ | $-(CH_2)_4-$ | 1 | H | $COOCH_3$ | $C_2H_5$ | $C_2H_5$ | $n_D^{25}$ | 1,5062 |
| 6 | $COOCH_3$ | $-(CH_2)_4-$ | 1 | H | $COOCH_3$ | $C_2H_5$ | $CH_2CH=CH_2$ | $n_D^{24}$ | 1,5124 |
| 7 | $COOCH_3$ | $-CH_2CH(CH_3)-$ | 1 | H | $COOCH_3$ | $C_2H_5$ | $C_2H_5$ | $n_D^{23}$ | 1,5030 |
| 8 | $COOCH_3$ | $-CH_2CH(CH_3)-$ | 1 | H | $COOCH_3$ | $C_2H_5$ | $CH_2CH=CH_2$ | $n_D^{23}$ | 1,5074 |
| 9 | $COOCH_3$ | $-CH_2CH(CH_3)-$ | 1 | H | $COOCH_3$ | n-$C_3H_7$ | $C_2H_5$ | $n_D^{22}$ | 1,5025 |
| 10 | $COOCH_3$ | $-CH_2CH(CH_3)-$ | 1 | H | $COOCH_3$ | n-$C_3H_7$ | $CH_2CH=CH_2$ | $n_D^{22}$ | 1,5070 |
| 11 | $COOCH_3$ | $-CH_2CH(CH_3)-$ | 1 | H | H | n-$C_3H_7$ | $C_2H_5$ | NMRs.u. | |
| 12 | CN | $-CH(CH_3)CH_2-$ | 1 | H | H | n-$C_3H_7$ | $CH_2CH=CHCl$ | $n_D^{23}$ | 1,5398 |
| 13 | CN | $-CH(CH_3)CH_2-$ | 1 | H | H | n-$C_3H_7$ | $C_2H_5$ | $n_D^{23}$ | 1,5213 |
| 14 | CN | $-CH(CH_3)CH_2-$ | 1 | H | H | n-$C_3H_7$ | $CH_2CH=CH_2$ | $n_D^{23}$ | 1,5271 |
| 15 | $COOC_2H_5$ | 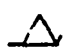 | 1 | H | $COOCH_3$ | n-$C_3H_7$ | $C_2H_5$ | $n_D^{24}$ | 1,5103 |
| 16 | $COOC_2H_5$ | 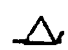 | 1 | H | $COOCH_3$ | n-$C_3H_7$ | $CH_2CH=CH_2$ | $n_D^{24}$ | 1,5144 |
| 17 | $CF_3$ | | 0 | $CH_3$ | H | n-$C_3H_7$ | $CH_2CH=CH_2$ | $n_D^{21,5}$ | 1,4829 |
| 18 | $CF_3$ | | 0 | $CH_3$ | H | n-$C_3H_7$ | $C_2H_5$ | $n_D^{21,5}$ | 1,4758 |
| 19 | $CF_3$ | | 0 | $CH_3$ | $COOCH_3$ | n-$C_3H_7$ | $CH_2CH=CH_2$ | $n_D^{22}$ | 1,4890 |
| 20 | $CF_3$ | | 0 | $CH_3$ | $COOCH_3$ | n-$C_3H_7$ | $C_2H_5$ | | |
| 21 | COOH | 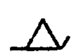 | 1 | H | H | n-$C_3H_7$ | $C_2H_5$ | NMR s.u. | |
| 22 | COOH | 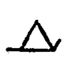 | 1 | H | H | n-$C_3H_7$ | $CH_2CH=CH_2$ | NMR s.u. | |
| 23 | $COOCH_3$ | $-CH_2CH(CH_3)(CH_2)_3-$ | 1 | H | $COOCH_3$ | n-$C_3H_7$ | $C_2H_5$ | $n_D^{29}$ | 1,4930 |
| 24 | $COOCH_3$ | $-CH_2CH(CH_3)(CH_2)_3-$ | 1 | H | $COOCH_3$ | n-$C_3H_7$ | $CH_2CH=CH_2$ | $n_D^{29}$ | 1,5000 |
| 25 | $COOCH_3$ | $-CH_2CH(CH_3)(CH_2)_3-$ | 1 | H | H | n-$C_3H_7$ | $C_2H_5$ | $n_D^{24}$ | 1,4972 |

| | | | | | | | | | |
|---|---|---|---|---|---|---|---|---|---|
| 26 | $COOCH_3$ | $-CH_2CH(CH_3)(CH_2)_3-$ | 1 | H | H | $n-C_3H_7$ | $CH_2CH=CH_2$ | $n_D^{24}$ | 1,5031 |
| 27 | $COOCH_3$ | $-CH_2CH(CH_3)(CH_2)_3-$ | 1 | H | H | $n-C_3H_7$ | $CH_2C\equiv CH$ | $n_D^{22}$ | 1,5090 |
| 28 | $COOC(CH_3)_3$ | $-CH(CH_3)-$ | 1 | H | H | $n-C_3H_7$ | $C_2H_5$ | $n_D^{23}$ | 1,4978 |
| 29 | $COOCH_3$ | $-CH_2CH(CH_3)(CH_2)_2CH(CH_3)-$ | 1 | H | H | $n-C_3H_7$ | $C_2H_5$ | NMR s.u. | |
| 30 | $COOCH_3$ | $-CH_2CH(CH_3)(CH_2)_2CH(CH_3)-$ | 1 | H | H | $n-C_3H_7$ | $CH_2CH=CH_2$ | NMR s.u. | |
| 31 | $CF_3$ | 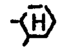 | 1 | H | H | $n-C_3H_7$ | $C_2H_5$ | NMR s.u. | |
| 32 | $CF_3$ | 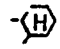 | 1 | H | H | $n-C_3H_7$ | $CH_2CH=CH_2$ | NMR s.u. | |
| 33 | $CF_3$ | 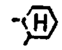 | 1 | H | H | $n-C_3H_7$ | $n-C_3H_7$ | NMR s.u. | |
| 34 | $CF_3$ | 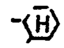 | 1 | H | H | $n-C_3H_7$ | $n-C_4H_9$ | | |
| 35 | $CF_3$ | 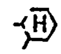 | 1 | H | H | $n-C_3H_7$ | $CH_2CH=CHCl$ (trans) | | |
| 36 | $CF_3$ | 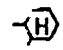 | 1 | H | H | $C_2H_5$ | $C_2H_5$ | | |
| 37 | $COOCH_3$ | $-(CH_2)_4-$ | 1 | H | H | $n-C_3H_7$ | $C_2H_5$ | NMR s.u. | |
| 38 | $COOCH_3$ | $-(CH_2)_4-$ | 1 | H | H | $n-C_3H_7$ | $CH_2CH=CH_2$ | | |
| 39 | $COOCH_3$ | $-CH_2CH(CH_3)-$ | 1 | H | H | $n-C_3H_7$ | $CH_3CH=CH_2$ (trans) | | |
| 40 | $COOCH_3$ | $-CH_2CH(CH_3)-$ | 1 | H | H | $n-C_3H_7$ | $CH_2CH=CH_2$ | | |
| 41 | $COOCH_3$ | $-CH_2CH(CH_3)-$ | 1 | H | H | $n-C_3H_7$ | $CH_2CH=CHCl$ | | |
| 42 | $COOCH_3$ | $-CH_2CH(CH_3)-$ | 1 | H | H | $C_2H_5$ | $C_2H_5$ | | |
| 43 | $CF_3$ | 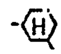 | 1 | H | H | $n-C_3H_7$ | $CH_2CH=CH_2$ | | |
| 44 | $CF_3$ | 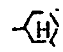 | 1 | H | H | $n-C_3H_7$ | $C_2H_5$ | | |
| 45 | $COOCH_3$ | 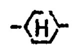 | 1 | H | H | $n-C_3H_7$ | $C_2H_5$ | | |
| 46 | $COOCH_3$ | 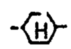 | 1 | H | H | $n-C_3H_7$ | $CH_2CH=CH_2$ | | |
| 47 | $COOCH_3$ | 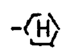 | 1 | H | H | $n-C_3H_7$ | $C_2H_5$ | | |
| 48 | $COOCH_3$ | 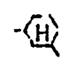 | 1 | H | H | $n-C_3H_7$ | $CH_2CH=CH_2$ | | |
| 49 | COOH | $-(CH_2)_4-$ | 1 | H | H | $C_2H_5$ | $CH_2CH=CHCl$ | NMR s.u. | |
| 50 | COOH | $-(CH_2)_4-$ | 1 | H | H | $C_2H_5$ | $CH_2CH=CHCl$ | NMR s.u. | |
| 51 | COOH | $-(CH_2)_4-$ | 1 | H | H | $n-C_3H_7$ | $C_2H_5$ | NMR s.u. | |
| 52 | COOH | $-(CH_2)_4-$ | 1 | H | H | $n-C_3H_7$ | $CH_2CH=CH_2$ | NMR s.u. | |
| 53 | COOH | $-(CH_2)_4-$ | 1 | H | H | $C_2H_5$ | $CH_2CH=CH_2$ | NMR s.u. | |
| 54 | $COOCH_3$ | $-(CH_2)_4-$ | 1 | H | H | $C_2H_5$ | $CH_2CH=CHCl$ (trans) | NMR s.u. | |
| 55 | $COOCH_3$ | $-(CH_2)_4-$ | 1 | H | H | $n-C_2H_5$ | $C_2H_5$ | NMR s.u. | |
| 56 | $COOCH_3$ | $-(CH_2)_4-$ | 1 | H | H | $n-C_3H_7$ | $CH_2CH=CHCl$ (trans) | NMR s.u. | |
| 57 | $COO-n-C_4H_9$ | $-(CH_2)_4-$ | 1 | H | H | $n-C_3H_7$ | $C_2H_5$ | NMR s.u. | |
| 58 | $COO-n-C_4H_9$ | $-(CH_2)_4-$ | 1 | H | H | $C_2H_5$ | $C_2H_5$ | NMR s.u. | |
| 59 | $COO-n-C_4H_9$ | $-(CH_2)_4-$ | 1 | H | H | $n-C_3H_7$ | $CH_2CH=CHCl$ (trans) | NMR s.u. | |
| 60 | $COO-n-C_4H_9$ | $-(CH_2)_4-$ | 1 | H | H | $C_2H_5$ | $CH_2CH=CHCl$ (trans) | NMR s.u. | |
| 61 | COOH | $-CH_2CH(CH_3)-$ | 1 | H | H | $C_2H_5$ | $C_2H_5$ | NMR s.u. | |
| 62 | $COO-n-C_4C_9$ | $-CH_2CH(CH_3)-$ | 1 | H | H | $C_2H_5$ | $C_2H_5$ | NMR s.u. | |
| 63 | COOH | $-CH_2CH(CH_3)-$ | 1 | H | H | $n-C_3H_7$ | $C_2H_5$ | NMR s.u. | |
| 64 | COOH | $-CH(C_2H_5)(CH_2)_2-$ | 1 | H | H | $C_2H_5$ | $C_2H_5$ | NMR s.u. | |
| 65 | $COOCH_3$ | $-(CH_2)_2CH(C_2H_5)-$ | 1 | H | H | $C_2H_5$ | $C_2H_5$ | | |
| 66 | $COOCH_3$ | $-(CH_2)_2CH(C_2H_5)-$ | 1 | H | H | $C_2H_5$ | $CH_2CH=CHCl$ (trans) | | |
| 67 | $COOCH_3$ | $-(CH_2)_2-$ | 1 | H | H | $CH_3$ | $C_2H_5$ | | |
| 68 | $COOCH_3$ | $-(CH_2)_2-$ | 1 | H | H | $C_2H_5$ | $C_2H_5$ | | |
| 69 | $COOCH_3$ | $-(CH_2)_2-$ | 1 | H | H | $n-C_3H_7$ | $C_2H_5$ | | |
| 70 | COOH | $-(CH_2)_2-$ | 1 | H | H | $n-C_3H_7$ | $C_2H_5$ | | |
| 71 | COOH | $-(CH_2)_2-$ | 1 | H | H | $C_2H_5$ | $C_2H_5$ | | |

| | | | | | | | |
|---|---|---|---|---|---|---|---|
| 72 | COOH | $-CH_2-$ | 1 | H | H | $C_2H_5$ | $C_2H_5$ |
| 73 | COOH | $-CH_2-$ | 1 | H | H | $C_2H_5$ | $CH_2-CH=CH_2$ |
| 74 | COOH | $-CH_2-$ | 1 | H | H | $n-C_3H_7$ | $C_2H_5$ |
| 75 | COOH | $-CH(CH_3)CH_2-$ | 1 | H | H | $C_2H_5$ | $C_2H_5$ |
| 76 | COOH | $-CH(CH_3)CH_2-$ | 1 | H | H | $n-C_3H_7$ | $C_2H_5$ |
| 77 | $COOCH_3$ | $-CH(CH_3)CH_2-$ | 1 | H | H | $C_2H_5$ | $C_2H_5$ |
| 78 | $COO-n-C_3H_7$ | $-CH(CH_3)CH_2-$ | 1 | H | H | $C_2H_5$ | $C_2H_5$ |

**Herstellung von Verbindung Nr. 102**

48,2 g 2-Butyryl-4-methoxycarbonyl-5-(tetramethylenmethoxycarbonyl)-cyclohexan-1,3-dion und 305 ml 10 gew.-%-ige wäßrige Kaliumhydroxidlösung wurden 48 Stunden bei Raumtemperatur gerührt, Man extrahierte mit Dichlormethan und säuerte die wäßrige Phase mit Salzsäure auf pH 1 an. Dann wurde auf 80° C erhitzt und bei dieser Temperatur 1 Stunde nachgerührt. Man ließ abkühlen, extrahierte zweimal mit Dichlormethan, trocknete die organische Phase über Natriumsulfat und destillierte das Lösungsmittel ab. Man erhielt 36 g 2-Butyryl-5-(tetramethylencarboxy)-cyclohexan-1,3-dion.

Die Substanz kann durch Umkristallisieren aus Petrolether gereinigt werden (Fp: 64° C).

Die in der folgenden Tabelle 2 aufgeführten Carbonylverbindungen der Formel II

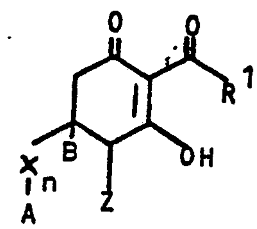

(II),

die als Zwischenprodukte für die Zielverbindungen der Formel I dienen, lassen sich auf analogem Weg herstellen.

Auch in dieser Tabelle soll bei den Substituenten X jeweils deren linke Bindung an den Cyclohexanring gebunden sein.

**Tabelle 2**

| Verbindung Nr. | A | X | n | B | Z | $R^1$ | phys. Daten |
|---|---|---|---|---|---|---|---|
| 79 | $CF_3$ | 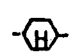 | 1 | H | H | $n-C_3H_7$ | NMR s.u. |
| 80 | $COOCH_3$ | $-(CH_2)_4-$ | 1 | H | $COOCH_3$ | $n-C_3H_7$ | NMR s.u. |
| 81 | $COOCH_3$ | $-(CH_2)_4-$ | 1 | H | $COOCH_3$ | $C_2H_5$ | NMR s.u. |
| 82 | $COOCH_3$ | $-CH_2CH(CH_3)-$ | 1 | H | $COOCH_3$ | $C_2H_5$ | NMR s.u. |
| 83 | $COOCH_3$ | $-CH_2CH(CH_3)-$ | 1 | H | $COOCH_3$ | $n-C_3H_7$ | NMR s.u. |
| 84 | $C\equiv N$ | $-CH(CH_3)CH_2-$ | 1 | H | H | $n-C_3H_7$ | NMR s.u. |
| 85 | $COOC_2H_5$ | 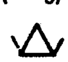 | 1 | H | $COOCH_3$ | $n-C_3H_7$ | NMR s.u. |
| 86 | $CF_3$ | | 0 | $CH_3$ | H | $n-C_3H_7$ | NMR s.u. |
| 87 | $CF_3$ | 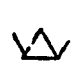 | 0 | $CH_3$ | $COOCH_3$ | $n-C_3H_7$ | NMR s.u. |
| 88 | COOH | | 1 | H | H | $n-C_3H_7$ | NMR s.u. |
| 89 | $COOCH_3$ | $-CH_2CH(CH_3)(CH_2)_3-$ | 1 | H | $COOCH_3$ | $n-C_3H_7$ | NMR s.u. |
| 90 | $COOCH_3$ | $-CH_2CH(CH_3)(CH_2)_3-$ | 1 | H | H | $n-C_3H_7$ | NMR s.u. |
| 91 | $COOC(CH_3)_3$ | $-CH_2CH(CH_3)-$ | 1 | H | H | $n-C_3H_7$ | NMR s.u. |
| 92 | $COOCH_3$ | $-CH_2CH(CH_3)(CH_2)_2CH(CH_3)-$ | 1 | H | H | $n-C_3H_7$ | NMR s.u. |
| 93 | $CF_3$ | 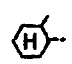 | 1 | H | H | $n-C_3H_7$ | |

| | | | | | | | |
|---|---|---|---|---|---|---|---|
| 94 | $CF_3$ | 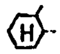 | 1 | H | H | $C_2H_5$ | |
| 95 | $COOCH_3$ | $-(CH_2)_4-$ | 1 | H | H | $n-C_3H_7$ | |
| 96 | $COOCH_3$ | $-CH_2CH(CH_3)-$ | 1 | H | H | $n-C_3H_7$ | |
| 97 | $COOCH_3$ | $-CH_2CH(CH_3)-$ | 1 | H | H | $C_2H_5$ | |
| 98 | $CF_3$ | 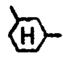 | 1 | H | H | $n-C_3H_7$ | |
| 99 | $COOCH_3$ | 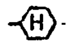 | 1 | H | H | $n-C_3H_7$ | |
| 100 | $COOCH_3$ | 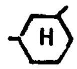 | 1 | H | H | $n-C_3H_7$ | |
| 101 | COOH | $-(CH_2)_4-$ | 1 | H | H | $C_2H_5$ | Fp.: 97 - 98°C |
| 102 | COOH | $-(CH_2)_4-$ | 1 | H | H | $n-C_3H_7$ | Fp.: 64°C |
| 103 | $COOCH_3$ | $-(CH_2)_4-$ | 1 | H | H | $C_2H_5$ | |
| 104 | $COO-n-C_4H_9$ | $-(CH_2)_4-$ | 1 | H | H | $n-C_3H_7$ | |
| 105 | $COO-n-C_4H_9$ | $-(CH_2)_4-$ | 1 | H | H | $C_2H_5$ | |
| 106 | COOH | $-CH_2CH(CH_3)-$ | 1 | H | H | $C_2H_5$ | NMR s.u. |
| 107 | $COO-n-C_4H_9$ | $-CH_2CH(CH_3)-$ | 1 | H | H | $C_2H_5$ | |
| 108 | COOH | $-CH_2CH(CH_3)-$ | 1 | H | H | $n-C_3H_7$ | NMR s.u. |
| 109 | COOH | $-CH(C_2H_5)(CH_2)_2-$ | 1 | H | H | $C_2H_5$ | NMR s.u. |
| 110 | $COOCH_3$ | $-(CH_2)_2CH(C_2H_5)-$ | 1 | H | H | $C_2H_5$ | |
| 111 | $COOCH_3$ | $-(CH_2)_2-$ | 1 | H | H | $CH_3$ | |
| 112 | $COOCH_3$ | $-(CH_2)_2-$ | 1 | H | H | $C_2H_5$ | |
| 113 | $COOCH_3$ | $-(CH_2)_2-$ | 1 | H | H | $n-C_3H_7$ | |
| 114 | COOH | $-(CH_2)_2-$ | 1 | H | H | $n-C_3H_7$ | |
| 115 | COOH | $-(CH_2)_2-$ | 1 | H | H | $C_2H_5$ | |
| 116 | COOH | $-CH_2-$ | 1 | H | H | $C_2H_5$ | NMR s.u. |
| 117 | COOH | $-CH_2-$ | 1 | H | H | $n-C_3H_7$ | |
| 118 | COOH | $-CH(CH_3)CH_2-$ | 1 | H | H | $C_2H_5$ | |
| 119 | COOH | $-CH(CH_3)CH_2-$ | 1 | H | H | $n-C_3H_7$ | |
| 120 | $COOCH_3$ | $-CH(CH_3)CH_2-$ | 1 | H | H | $C_2H_5$ | NMR s.u. |
| 121 | $COO-n-C_3H_7$ | $-CH(CH_3)CH_2-$ | 1 | H | H | $C_2H_5$ | |

**NMR-Daten**

| Verbindung Nr. (aus Tabelle 1) | | $^1H$-NMR-Daten (in $CDCL_3$/TMS) [δ] | |
|---|---|---|---|
| 11 | 0,95 (t) | 1,35 (t) | 3,7 (s) |
| 21 | 0,95 (t) [3 Prot] | 1,35 (t) [3] | 2,95 (t) [2] |
| | 4 15 (g) [2] | | |
| 22 | 0,95 (t) [3] | 2,95 (t) [2] | 4,55 (d) [2] |
| | 5,35 (m) [2] | 6,0 (m) [1] | |
| 29 | 2,95 (t) [3] | 3,65 (t) [3] | 4,10 (g) [2] |
| 30 | 1,2 (m) | 3,70 (s) [3] | 4,55 (d) [2] |
| 31 | 1,30 (t) [3] | 4,11 (g) [2] | |
| 32 | 4,52 (d) [2] | 5,39 (m) [2] | 6,01 (m) [1] |
| 33 | 0,97 (t) [3] | 1,76 (m) [2] | 4,03 (t) [2] |
| 34 | 0,98 (t) [3] | 1,43 (m) [2] | 1,70 (m) [2] |
| | 4,06 (t) [2] | | |
| 35 | 4,54 (d) [2] | 6,12 (m) [1] | 6,37 (d) [1] |
| 37 | 0,9 (t) | 2,3 (t) | 4,1 (q) |
| 49 | 1,4 (s) | 2,4 (t) | 6,1 (m) |
| 50 | 1,1 (t) | 1,3 (t) | 2,9 (q) |
| 51 | 0,95 (t) | 2,9 (t) | 4,1 (q) |
| 52 | 0,95 (t) | 1,4 (s) | 4,55 (d) |
| 53 | 1,4 (s) | 1,6 (m) | 2,4 (t) |
| 54 | 1,4 (s) | 3,7 (s) | 4,5 (d) |
| 55 | 1,1 (t) | 1,3 (t) | 4,1 (q) |
| 56 | 0,95 (t) | 2,3 (t) | 3,65 (s) |
| 57 | 0,95 (t) | 2,3 (t) | 2,9 (t) |
| 58 | 0,95 (t) | 1,3 (t) | 2,5 (m) |
| 59 | 1,4 (d) | 2,3 (t) | 4,1 (m) |
| 60 | 1,15 (t) | 2,3 (t) | 4,5 (d) |

| | | | |
|---|---|---|---|
| 61 | 1,1 (t) | 2,9 (q) | 4,1 (q) |
| 62 | 0,9 (t) | 1,4 (t) | 2,5 (m) |
| 63 | 1,3 (d) | 2,2 (m) | 4,1 (q) |
| 64 | 0,9 (t) | 1,15 (t) | 2,9 (q) |
| (aus Tabelle 2) | | | |
| 79 | 0,98 (t) | 3,0 (t) | |
| 80 | 0,92 (t) | 2,95 (t) | 3,65 (s) |
| 81 | 1,17 (t) | 2,35 (t) | 3,83 (s) |
| 82 | 1,13 (t) | 3,05 (q) | 3,70 (s) |
| 83 | 0,93 (t) | 3,7 (s) | 3,8 (s) |
| 84 | 0,98 (t) | 1,13 (d) | 2,97 (t) |
| 85 | 1,27 (t) | 1,65 (q) | 3,7 (s) |
| 86 | 1,05 (t) | 1,35 (s) | 3,05 (t) |
| 87 | 1,4 (s) | 1,65 (q) | 3,8 (s) |
| 88 | 1,0 (t) | 3,0 (t) | |
| 89 | 0,9 (t) | 3,6 (s) | 3,7 (s) |
| 90 | 1,0 (t) | 3,1 (t) | 3,7 (s) |
| 91 | 0,95 (t) | 1,4 (s) | 3,0 (t) |
| 106 | 1,3 (m) | 2,5 (m) | 3,1 (q) |
| 108 | 1,0 (t) | 3,0 (q) | |
| 109 | 0,90 (t) | 1,1 (t) | 3,1 (q) |

Die Cyclohexenonderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubermitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, Öle pflanzlichen oder tierischen Ursprungs, aliphatische, cyclische und aromatische Kohlenwasserstoffe, wie Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Heft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalindervaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Kalkstein, Kalk, Kreide Bolus, Löß, Ton, Dolomit, Diatomeenderde, Calcium- und Magnesiumsulfat oder Magnesiumoxid, gemahlene Kunststoffe, Düngemittel, wie z. B. Ammoniumsulfat, Ammoniumphosphat oder Ammoniumnitrat, Harnstoffe und pflanzliche Produkte, wie Getreidemehl, Baumrinden-, Holz- und Nußschalemehl, Cellulosepulver und andere feste Trägerstoffe.

Die Formulierungen enthalten zwischen 0,1 und 95 Gew.-%, vorzugsweise zwischen 0,5 und 90 Gew.-%, Wirkstoff.

Beispiele für Formulierungen sind:

I. Man vermischt 90 Gew.-Teile der Verbindung Nr. 12 mit 10 Gew.-Teilen N-Methyl-alpha-pyrrlidon und erhält eine Lösung, die zur Anwendung in Form kleinster Tropfen geeignet ist.

II. 20 Gew.-Teile der Verbindung Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teile Calciumsalz der Dodecylbenzolsulfonsäure und 5 Gew.-Teile des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.

III. 20 Gew.-Teile der Verbindung Nr. 2 werden in einer Mischung gelöst, die aus 40 Gew.-Teilen Cyclohexanon, 30 Gew.-Teilen Isobutanol, 20 Gew.-Teile des Anlagerungsproduktes von 7 Mol Ethylenoxid an 1 Mol Isooctylphenol und 10 Gew.-Teile des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

IV. 20 Gew.-Teile des Wirkstoffs Nr. 25 werden in einer Mischung gelöst, die aus 25 Gew.-Teilen Cyclohexanol, 65 Gew.-Teile einer Mineralölfraktion vom Siedepunkt 210 bis 280°C und 10 Gew.-Teilen des Anlagerungsproduktes von 40 Mol Ethylenoxid an 1 Mol Ricinusöls besteht. Ourch Eingießen und feines Verteilen der Lösung in 100 000 Gew.-Teilen Wasser erhält man eine wäßrige Dispersion, die 0,02 Gew.-% des Wirkstoffs enthält.

V. 20 Gew.-Teile des Wirkstoffs Nr 13 werden mit 3 Gew.-Teilen des Natriumsalzes der Diisobutylnaphthalin-alpha-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.

VI. 3 Gew.-Teile des Wirkstoffs Nr. 17 werden mit 97 Gew.-Teilen feinteiligem Kaolin vermischt. Man erhält auf diese Weise ein Stäubemittel, das 3 Gew.-% des Wirkstoffs enthält.

VII. 30 Gew.-Teile des Wirkstoffs Nr. 19 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. man erhält auf diese Weise eine Aufbereitung des Wirkstoffs mit guter Haftfähigkeit.

VIII. 20 Teile des Wirkstoffs Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensetes und 68 Teile eines paraffinischen Mineralöls innig vermischt. Men erhält eine stabile ölige Dispersion.

Die Applikation kann im vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritz werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,025 bis 3 kg/ha, vorzugsweise 0,06 bis 0,5 kg/ha.

Die Wirkung der Cyclohexenolderivate der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem aufbringen der Mittel werden die Gefäße leicht beregnet, um Keimung und Wachstum in Gang zu bringen. Danach deckt man die Gefäße mit durchsichtigen Ptastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zweck der Nachauflaufbehandlung zieht man die Testpflanzen je nach Wuchsform erst bis zu einer Wuchshöhe von 3 bis 15 cm an und behandelte sie danach, Die Sojaplanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung betragen 0,06 bis 0,5 Wirkstoff/ha. Eine Abdeckung unterbleibt bei der Nachauflaufbehandlung.

Die Versuchgefäße werden im Gewächshaus aufgestellt, wobei für wärmelieben Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen verpflegt und ihre Reaktion auf die einzelnen Behandlungen wird ausgewertes. Bewertet wird nach einer Skala von 0 bis 100. Dabei bedeutet 100 kein Aufgang der Pflanzen bzw. völlig Zerstörung zumindest der oberirdischen Teile.

Die in den Gewächshausversuchen verwendeten Pflanzen setzen sich aus folgenden Arten zusammen:

Alopecurus myosuroides (Ackerfuchsschwanz)

| | |
|---|---|
| Avena fatua | (Flughafer) |
| Avena sativa | (Hafer) |
| Digitaria sanguinalis | (Blutfingerhirse) |
| Echinochloa crus-galli | (Hühnerhirse) |
| Clycine max | (Sojabohnen) |
| Lolium multiflorum | (Ital. Raygrass) |
| Medicago sativa | (Luzerne) |
| Setaria italica | (Kolbenhirse) |
| Sinapis alba | (Weißer Senf) |
| Sorghum halepense | (Wilde Mohrenhirse) |
| Triticum aestivum | (Weizen) |
| Zea mays | (Mais) |

Bei Vorauflaufanwendung erweisen sich die beispielhaft ausgewählten Verbindungen Nr. 1, 2, 12, 13, 14, 17, 19 und 25 als herbizid wirksam gegen Pflanzen aus der Familie der Gräser, während Sinapis alba als Vertreter dikotyler Pflanzen völlig ungeschädigt bleibt.

Weiterhin zeigen beispielsweise die Verbindungen Nr. 12 und 14 mit 0,25 kg Wirkstoff/ha bei Nachauflaufbehandlung starke herbizide Aktivität gegen Gräser, während Sojabohnen als dikotyle Kulturpflanze keinen Schaden erleiden. Mit 0,5 kg Wirkstoff/ha der Wirkstoffe Nr. 1 und 2 lassen sich in Sojabohnen Ausfallsmais und Setaria italica als Beispielspflanze für Hirsen selektiv bekämpfen. Auch in einer Gramineenkultur, wie beispielsweise Weizen können die Cyclohexenonderivate der Formel I als Gräserherbizide eingesetzt werden. Verbindung Nr, 25 eignet sich z. B. zur Bekämpfung wichtiger Ungräser bei einer Aufwandmenge von 0,125 kg Wirkstoff.

Ebenfalls im Nachauflaufverfahren zeigen die Verbindungen Nr. 12 und 14 bei geringen Aufwandmengen eine starke herbizide Wirkung gegen weitverbreitete Schadgräser, wie Alopecurus myosuroides und Avena fatua.

Die beispielhaft ausgewählte Verbindung Nr. 32 eignet sich gut zur Bekämpfung eines breiten Gräserspektrums in breitblättrigen Kulturen. An Soja wird dabei beispielhaft gezeigt, daß die Kulturpflanze keinen Schaden erleidet.

In Anbetracht des erfaßbaren Wirkungsspektrums zur Unkrautbekämpfung, der Verträglichkeit für Kulturpflanzen oder der unerwünschten Beeinflussung des Wachstums derselben sowie angesichts der Vielfalt der Applikationsmethoden können die Cyclohexenonderivate der Formel I in einer großen Zahl von Kulturpflanzen eingesetzt werden. In Betracht kommen beispielsweise folgende Kulturen:

| Botanischer Name | Dutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |

| | |
|---|---|
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triszinone, Uracile, Benzofuranderivate, Chinolin-carbonsäuren und andere in Betracht.

Außerdem kann es von Nutzen sein, die Cyclohexenonderivate der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. Cyclohexenonderivate der Formel I

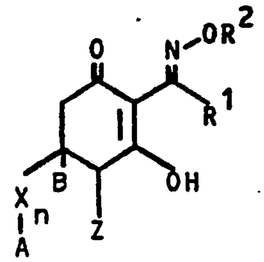

(I),

in der
$R^1$ $C_1$-$C_4$-Alkyl,
$R^2$ $C_1$-$C_4$-Alkyl, gegebenenfalls halogensubstituiertes $C_3$-$C_5$-Alkenyl oder $C_3$-$C_5$-Alkinyl,
A $C_2$-$C_5$-Alkoxycarbonyl, Carboxyl, Cyano oder Trifluormethyl,
B Wasserstoff oder Methyl,
X einen unverzweigten, verzweigten oder cyclischen Alkylenrest mit bis zu 7 C-Atomen,
Z Wasserstoff oder $C_2$-$C_5$-Alkoxycarbonyl und
n 0 oder 1 bedeuten, mit der Maßgabe, daß n nicht 0 bedeutet, wenn A für Alkoxycarbonyl oder Cyano steht, und Salze dieser Verbindungen.

2. Verfahren zur Herstellung eines Cyclohexenonderivates der Formel I gemäß Anspruch 1, dadurch gekennzeichnet, daß man eine Carbonylverbindung der Formel II

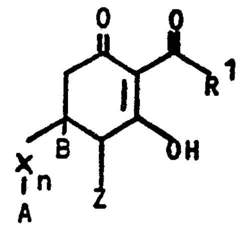

(II),

in der $R^1$, A, B, Z, X und n jeweils die in Anspruch 1 genannte Bedeutung haben,

a) mit einer Ammoniumverbindung der Formel $R^2ONH_3Y$, in der $R^2$ die in Anspruch 1 genannte Bedeutung hat und Y ein Anion bedeutet, in einem inertan Verdünnungsmittel gegebenenfalls in Gegenwart einer Base

oder

b) mit einem gegebenenfalls in wäßriger Lösung vorliegenden Hydroxylamin dar Formel $R^2ONH_2$, in der $R^2$ die in Anapruch 1 genannte Bedeutung hat, gegebenenfalls in Gegenwart eines Verdünnungsmittels,

oder

c) mit unsubstituiertem Hydroxylammoniumsalz der Formel $HONH_3Y$, in der Y ein Anion bedeutet. in Gegenwart eines Lösungsmittels und einer Base umsetzt und das so erhaltene Oxim mit einem Alkylierungsmittel der Formel $R^2Y'$, in der $R^2$ die in Anspruch 1 genannte Bedeutung hat und Y' eine Abgangsgruppe bedeutet,

umzetzt.

3. Herbizid, enthaltend ein Cyclohexanonderivat der Formel I gemäß Anspruch 1.

4. Herbizid, enthaltend inerte Zusatzstoffe und ein Cyclohexenonderivat der Formel I gemäß Anspruch 1.

5. Verfahren zur Bekämpfung unerwünschten Pflanzenwuchses, dadurch gekennzeichnet, daß man die unerwünschten Pflanzen oder von unerwünschtem Pflanzenwuchs freizuhaltende Fläche mit einer herbizid wirksamen Menge eines Cyclohexenonderivetes der Formel I gemäß Anspruch 1 behandelt.

6. Carbonylverbindungen der Formel II

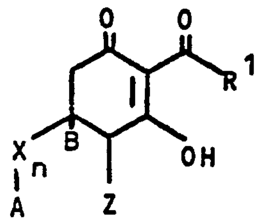

(II),

in der
$R^1$ $C_1$-$C_4$-Alkyl,
A $C_2$-$C_5$-Alkoxycarbonyl, Carboxyl, Cyano oder Trifluormethyl,
B Wasserstoff oder Methyl,
X einen unverzweigten, verzweigten oder cyclischen Alkylenrest mit bis zu 7 C-Atomen,
Z Wasserstoff oder $C_2$-$C_5$-Alkoxycarbonyl und
n 0 oder 1 bedeuten, mit der Maßgabe, daß n nicht 0 bedeutet, wenn A für Alkoxycarbonyl oder Cyano steht.

**Claims**

1. A cyclohexenone derivativ of the formula I

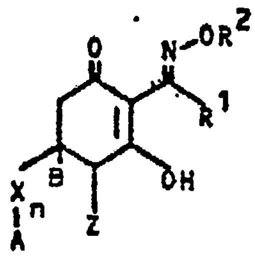

(I),

where
$R^1$ is $C_1$-$C_4$-alkyl,
$R^2$ is $C_1$-$C_4$-alkyl, unsubstituted or halogen-substituted $C_3$-$C_5$-alkenyl or $C_3$-$C_5$-alkynyl,
A is $C_2$-$C_5$-alkoxycarbonyl, carboxyl, cyano or trifluarmethyl,
B is hydrogen or methyl,
X is straight-chain, branched or cyclic alkylene of not more than 7 carbon atoms,
Z is hydrogen or $C_2$-$C_5$-alkoxy-carbonyl and
n is 0 or 1, with the proviso that n is not O when A is alkoxycarbonyl or cyano, or a salt thereof.

2. A process for the preparation of a cyclohexenone derivative of the formula I as claimed in claim 1, wherein a carbonyl compound of the formula II

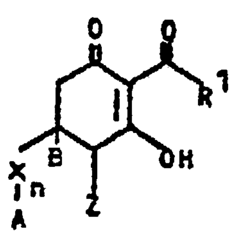

(II),

where $R^1$, A, B, Z, X and n are each as defined in claim 1, is reacted

a) with an ammonium compound of the formula $R^2ONH_3Y$, where $R^2$ is as defined in claim 1 and Y is an anion, in an inert diluent in the presence or absence of a base or
b) with a hydroxylamine of the formula $R^2ONH_2$ which may be in aqueous solution, and in which $R^2$ is as defined in claim 1, in the presence or absence of a diluent or

c) with an unsubstituted hydroxylammonium salt of the formula $HONH_3Y$, where Y is an anion, in the presence of a solvent and of a base and the oxime thus obtained is reacted with an akylating agent of the formula $R^2Y'$, where $R^2$ is as defined in claim 1 and Y' is a leaving group.

3. A herbicide containing a cyclohexenone derivative of the formula I as claimed in claim 1.

4. A herbicide containing inert additives and a cyclohexenone derivative of the formula I as claimed in claim 1.

5. A process for combating unwanted plant growth, wherein the unwanted plants or the area to be kept free from unwanted plant growth is treated with a herbicidally effective amount of a cyclohexenone derivative of the formula I as claimed in claim 1.

6. A carbonyl compound of the formula II

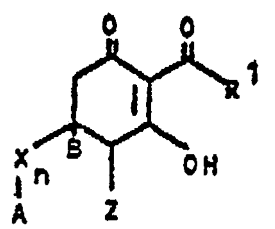

(II),

where

$R^1$ is $C_1$-$C_4$-alkyl,

A is $C_2$-$C_5$-alkoxy-carbonyl, carboxyl, cyano or trifluoromethyl,

B is hydrogen or methyl,

X is straight-chain, branched or cyclic alkylene of not more than 7 carbon atoms,

Z is hydrogen or $C_2$-$C_5$-alkoxycarbonyl, and

n is 0 or 1, with the proviso that n is not 0 when A is alkoxycarbonyl or cyano.

**Revendications**

1. Dérivés de cyclohexenone de formule I

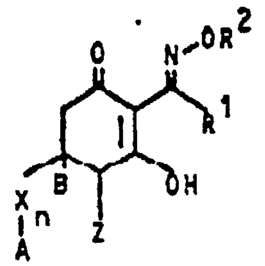

(I),

dans laquelle

$R^1$ représente un alkyleen $C_1$-$C_4$,

$R^2$, alkyle en $C_1$-$C_4$, alcényle en $C_3$-$C_5$ ou alcynyle en $C_3$-$C_5$ éventuellement substitués par un haloqène

A, alcoxycarbonyle en $C_2$-$C_5$, carboxyle, cyano ou trifluorométhyle,

B, hydrogène ou méthyle

X, un reste alkylène, ramifié, non ramifié ou cyclique, ayant jusqu'à 7 atomes C

Z, hydrogène ou alcoxycarbonyle en $C_2$-$C_5$, et

n, 0 ou 1

à condition que n ne représente pas 0 quand A représente alcoxycarbonyle ou cyano, et sels de ces dérivés.

2. Procédé de préparation d'un dérivé de cyclohexenone de formule I selon la revendication 1, caractérisé par le fait qu'on fait réagir un composé carbonyle de formule II

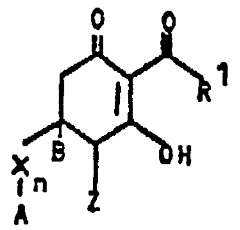

(II),

dans laquelle $R^1$, A, B, Z, X et n ont les siqnifications indiquées dans la revendication 1,

a) avec un composé d'ammonium de formule $R^2ONH_3Y$, dans laquelle $R^2$ a la signification indiquée dans la revendication 1 et Y représente un anion, dans un diluant inerte, éventuellement en présence d'une base

ou

b) avec une hydroxylamine de formule $R^2ONH_2$, se trouvant éventuellement en solution aqueuse, ou $R^2$ a la signification donnée dans la revendication 1, éventuellement en présence d'un diluant,

ou

c) avec un sol d'hydroxylammonium, non substitué, de formule $HONH_3Y$, dans laquelle Y représente un anion, en présence d'un solvant et d'une base, et on fait réagir l'oxime ainsi obtenu avec un agent d'alkylation de formule $R^2Y'$, dans laquelle $R^2$ a la signification indiquée dans la revendication 1 et Y' représente un groupe éliminable.

3. Herbicide contenant un dérive de cyclohexenone de formule I selon la revendication 1.

4. Herbicide contenant des additifs inertes et un dérivé de cyclohexenone de formule I selon la revendication 1.

5. Procédé pour lutter contre la croissance de plantes indésirables, caractérisé par le fait que l'on traite les plantes indésirables ou les surfaces à maintenir libres d'une croissance de plantes indésirables avec une quantité efficace du point de vue herbicide d'un dérivé de cyclohexenone de formule I selon la revendication 1.

6. Dérivés carbonylés de formule II

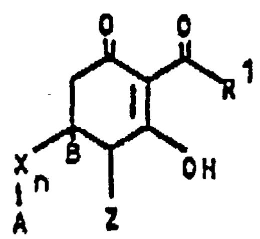

(II),

dans laquelle

$R^1$ représente alkyle en $C_1$-$C_4$

A, alcoxycarbonyle en $C_2$-$C_5$, carbonyle, cyano ou trifluorométhyle,

B, hydrogène ou méthyle

X, un reste alkylène, ramifié, non ramifié ou cyclique, ayant jusqu'à 7 atomes C

Z, hydrogène ou alcoxycarbonyle en $C_2$-$C_5$ et

n, 0 ou 1

à condition que n ne représente pas 0 quand A représente alcoxycarbonyle ou cyano.